(19) Europäisches Patentamt
European Patent Office
Office européen des brevets

(11) Veröffentlichungsnummer: **0 154 273**
**B1**

(12) **EUROPÄISCHE PATENTSCHRIFT**

(45) Veröffentlichungstag der Patentschrift: **13.09.89**

(21) Anmeldenummer: **85102041.2**

(22) Anmeldetag: **25.02.85**

(51) Int. Cl.⁴: **A 01 N 43/80**, C 07 D 275/06 // C07D417/12

# (54) Fungizide Mittel.

(30) Priorität: **08.03.84 DE 3408540**

(43) Veröffentlichungstag der Anmeldung: **11.09.85 Patentblatt 85/37**

(45) Bekanntmachung des Hinweises auf die Patenterteilung: **13.09.89 Patentblatt 89/37**

(84) Benannte Vertragsstaaten: **AT BE CH DE FR GB IT LI NL**

(56) Entgegenhaltungen:
**EP-A-0 086 748**
**US-A-4 178 451**

**JOURNAL OF MEDICINAL CHEMISTRY, Band 10, Nr. 5, September 1967, Seiten 844-849, Washington, DC, US; CALVERT W., WHITEHEAD et al.: "Hypotensive 1,2-Benzisothiazole 1,1-Dioxides. II. 3-Hydrazino-1,2-benzisothiazole 1,1-Dioxides and their Hydrazone and Amide Derivatives"**

(73) Patentinhaber: **BAYER AG, D-5090 Leverkusen 1 Bayerwerk (DE)**

(72) Erfinder: **Salzburg, Herbert, Dr., Hahnenweg 1, D-5000 Köln 80 (DE)**
Erfinder: **Hajek, Manfred, Dr., Hahnenweg 3, D-5000 Köln 80 (DE)**
Erfinder: **Hänssler, Gerd, Dr., Heymannstrasse 40, D-5090 Leverkusen (DE)**

**EP 0 154 273 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft die Verwendung von größtenteils bekannten 3-Hydrazino-1,2-benzisothiazol-1,1-dioxid-Derivaten als Fungizide. Die Verwendung der 3-Hydrazino-1,2-benzisothiazol-1,1-dioxid-Derivate in der Pharmakologie ist bekannt (vgl. Whitehead u. a., J. Med. Chem. 10, 844 ff., (1967)). Ihre Verwendung auf dem Pflanzenschutzgebiet, vor allem als Fungizide ist neu.

Weiterhin sind 3-Alkenyloxy-1,2-benzisothiazol-1,1-dioxide, z. B. das 3-Allyloxy-1,2-benzisothiazol-1,1-dioxid, bekannt, ebenso ihre Wirkung auf dem Pflanzenschutzgebiet (vgl. Jap. Pat. 7 014 301; CA 73: 45 500 m).

Es wurde gefunden, daß die als Pharmaka bekannten 3-Hydrazino-1,2-benzisothiazol-1,1-dioxid-Derivate der allgemeinen Formel (I)

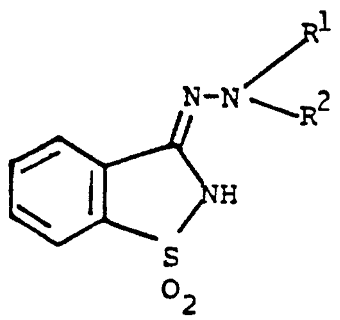

(I)

in welcher

$R^1$ für Wasserstoff, für Alkyl mit 1 bis 4 Kohlenstoffatomen, für Cycloalkyl mit 5 oder 6 Kohlenstoffatomen oder für Phenyl steht,

$R^2$ für -CO-$R^3$ steht, wobei

$R^3$ für Alkyl mit 1 bis 4 Kohlenstoffatomen, für Cyclohexyl, für Benzyl, für Phenyl, für Alkoxy mit 1 bis 4 Kohlenstoffatomen, für Phenoxymethyl, für gegebenenfalls ein- bis dreifach durch Halogen substituiertes Phenoxy, für Alkylamino mit 1 bis 4 Kohlenstoffatomen, für Cycloalkylamino mit 5 oder 6 Kohlenstoffatomen oder für Phenylamino steht, oder

$R^1$ und $R^2$ gemeinsam für eine

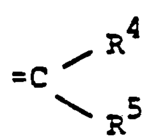

-Gruppe stehen,

worin

$R^4$ für Wasserstoff, oder für Alkyl mit 1 bis 3 Kohlenstoffatomen steht,

$R^5$ für Alkyl mit 1 bis 4 Kohlenstoffatomen, für Alkenyl mit 2 bis 5 Kohlenstoffatomen, für Cycloalkenyl mit 5 oder 6 Kohlenstoffatomen, für Phenylvinyl, für Furyl, für gegebenenfalls ein- bis dreifach durch Halogen, insbesondere Fluor und Chlor, Alkyl mit 1 bis 3 Kohlenstoffatomen und Alkoxy mit 1 bis 3 Kohlenstoffatomen substituiertes Phenyl steht, oder

$R^4$ und $R^5$ für Alkylen mit 4 oder 5 Kohlenstoffatomen stehen,

gute fungizide Eigenschaften besitzen.

Überraschenderweise zeigen die 3-Hydrazino-1,2-benzisothiazol-1,1-dioxid-Derivate der Formel (I) eine höhere fungizide, insbesondere systemische Wirkung als die aus dem Stand der Technik bekannten Verbindungen gleicher Wirkungsrichtung. Die erfindungsgemäße neue Verwendung der 3-Hydrazino-1,2-benzisothiazol-1,1-dioxid-Derivate stellt somit eine Bereicherung der Technik dar.

Die erfindungsgemäß zu verwendenden 3-Hydrazino-1,2-benzisothiazol-1,1-dioxid-Derivate sind durch die Formel (I) allgemein definiert.

Die Verbindungen der Formel (I) liegen im allgemeinen im Gleichgewicht mit den Verbindungen der Formel (IA) vor:

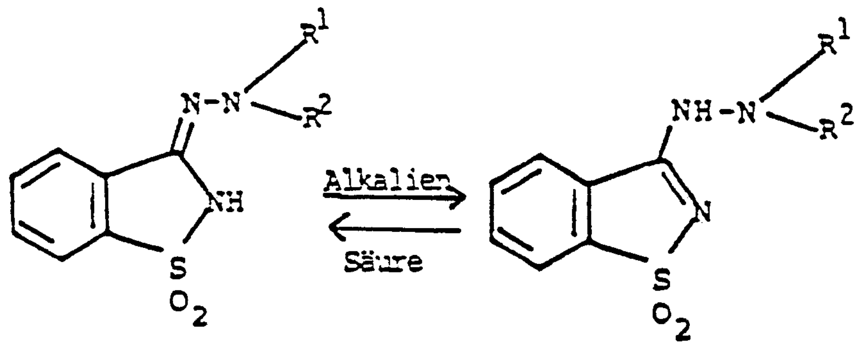


(I) (IA)

Im nachfolgenden wird der Einfachheit halber stets von Verbindungen der Formel (I) gesprochen, obwohl die reinen Verbindungen oder ihre Gemische mit unterschiedlichen Anteilen der Verbindungen der Formel (I) und (IA) gemeint sind.

Besonders bevorzugt sind die Verbindungen der Formel (I), bei denen
$R^1$ für Wasserstoff oder für Methyl steht,
$R^2$ für $-CO-R^3$ steht, wobei
$R^3$ für Methyl, Ethyl, n- und iso-Propyl, für Phenyl, für Benzyl, für Phenoxymethyl, für Methoxy, Ethoxy und n- und iso-Propoxy, für gegebenenfalls ein- oder zweifach durch Chlor substituiertes Phenoxy, für Methylamino, Ethylamino, n- und iso-Propylamino, für Cyclohexylamino oder Phenylamino steht, oder
$R^1$ und $R^2$ gemeinsam für eine

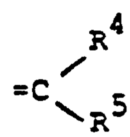

-Gruppe stehen,
worin
$R^4$ für Wasserstoff, für Methyl oder Ethyl steht,
$R^5$ für Methyl, Ethyl, n- und iso-Propyl, für Ethenyl, 1-Methyl-ethenyl, Allyl, Propenyl, 1-Methyl-propenyl, für Cyclohexenyl, für Phenylvinyl, für Furyl, für gegebenenfalls ein- oder zweifach durch Fluor, Chlor, Methyl, Ethyl, Methoxy und Ethoxy substituiertes Phenyl steht, oder
$R^4$ und $R^5$ für Tetra- oder Pentamethylen stehen.

Die erfindungsgemäß zu verwendenden 3-Hydrazino-1,2-benzisothiazol-1,1-dioxid-Derivate der Formel (I) sind größtenteils bekannt. Man kann sie nach bekannten Verfahren herstellen (vgl. z. B. Whitehead u. a., J. Med. Chem. 10, 844 - 849 (1967) oder J. Am. Chem. Soc. 65, 457 - 458 (1943)). So kann man das 3-Hydrazino-2H,3H-1,2-benzisothiazol-1,1-dioxid mit aktivierten Carbonsäurederivaten, wie Anhydriden und Halogeniden, umsetzen gemäß dem folgenden Formelschema:

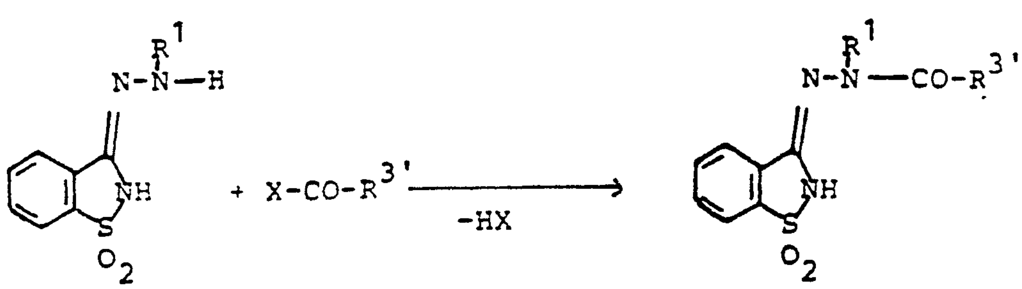


wobei
$R^1$ die oben angegebene Bedeutung hat,
$R^{3'}$ die oben für $R^3$ angegebene Bedeutung hat ausgenommen die Amidderivate und
X für Halogen oder den $-O-CO-R^{3'}$-Rest steht.

Die Reaktion wird üblicherweise bei Normaldruck und einer Temperatur von 0°C bis 100°C durchgeführt, gegebenenfalls in Anwesenheit einer Hilfsbase für die Reaktion mit Halogeniden und in Gegenwart inerter Lösungsmittel, wie z. B. Dioxan, Chloroform, Methylenchlorid, Toluol nach den Standard-Verfahren der

organischen Chemie.

Auch die Verbindungen der Formel (I), in der $R^1$ und $R^2$ gemeinsam für eine

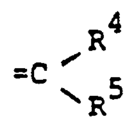

-Gruppe stehen,

können nach bekannten Verfahren hergestellt werden (vgl. Whitehead u. a., J. Med. Chem. 10, 844 - 849 (1967)). Man setzt das 3-Hydrazino-1,2-benzisothiazol-1,1-dioxid mit Oxoverbindungen nach folgendem Formelschema um:

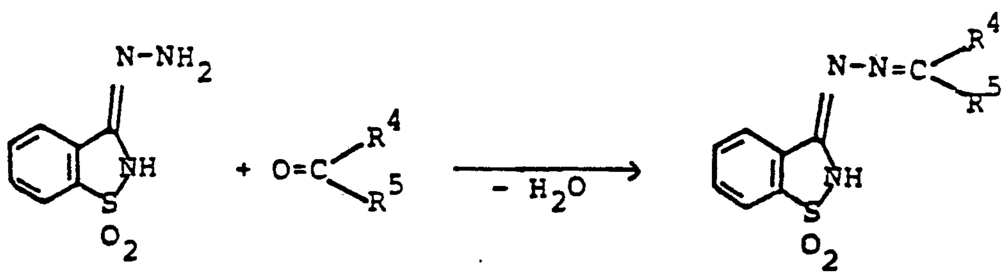


wobei

$R^4$ und $R^5$ die oben angegebene Bedeutung haben. Im allgemeinen setzt man die Reaktionskomponenten in stöchiometrischen Mengenverhältnissen in einem geeignetem Lösungsmittel unter Erwärmen um. Geeignete Lösungsmittel sind z. B. Dioxan, Ethanol, Dimethylformamid und Toluol.

Das Verfahren wird normalerweise bei Normaldruck durchgeführt, wobei die Temperatur zwischen 10°C und 120°C, in der Regel bei 80 - 100°C liegt.

Bevorzugte erfindungsgemäße Oxoverbindungen sind Aldehyde, wie z. B. Acetaldehyd, Butyr- und Isobutyraldehyd, Benzaldehyd, 3-Chlorbenzaldehyd, 3,4-Dichlorbenzaldehyd, 4-Chlorbenzaldehyd, 4-Methoxybenzaldehyd, 3- und 4-Fluorbenzaldehyd, Acrolein, Methacrolein, 2-Furylaldehyd, 2-Methylpropenylaldehyd, $\Delta^3$-Cyclohexenylaldehyd, Indolylaldehyd, Styrylaldehyd.

Bevorzugte Ketone sind z. B. Aceton, Cyclopentanon, Cyclopentenon, Cyclohexanon, Methylethylketon.

Die Verbindungen der Formel (IB)

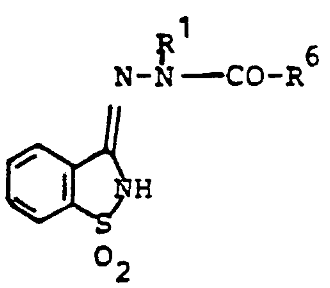

(IB).

in welcher

$R^1$ die in der Formel (I) angegebene Bedeutung hat und

$R^6$ für Alkylamino mit 1 bis 4 Kohlenstoffatomen, für Cycloalkylamino mit 5 oder 6 Kohlenstoffatomen oder Phenylamino steht, ausgenommen die Verbindung in der $R^1$ für Wasserstoff und $R^6$ für Phenylamino steht (vgl. Whitehead, J. Med. Chem. 10, 844 ff. (1967)),

sind neu; sie können aber nach altbekannten Methoden der klassischen organischen Chemie hergestellt werden, indem man z. B. 3-Hydrazino-2H,3H-1,2-benzisothiazol-1,1-dioxide der Formel (II)

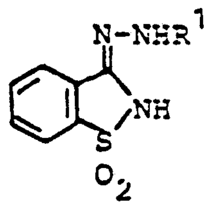

(II)

in welcher
$R^1$ die oben angegebene Bedeutung hat, mit Isocyanaten der Formel (III)

$OCNR^6$ (III)

in welcher
$R^6$ die oben angegebene Bedeutung hat, gegebenenfalls in Gegenwart eines inerten Lösungsmittels, wie oben bereits genannt, vorzugsweise bei Normaldruck, meist in äquimolarem Verhältnis umsetzt.

Die erfindungsgemäß verwendbaren Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet, vor allem als Fungizide, einige Verbindungen sind auch bakterizid wirksam.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z. B. nichtionogene und anionische Emulgatoren, wie Polyoxyethylen-Fettsäureester, Polyoxyethylen-Fettalkoholether, z. B. Alkylarylpolyglycol-Ether, Alkylsulfonate, Alkylsulfate, Arylsulfonate sowie Eiweißhydrolysate. Als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Selektiv-Herbizide, sowie in Mischungen mit Düngemittel und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

**Anwendungsbeispiele**

Die nachfolgend aufgeführte Verbindung wird als Vergleichssubstanz in den Testbeispielen verwendet.

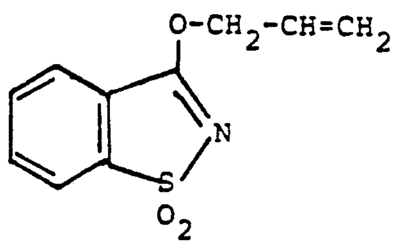

**Beispiel A**

Pyricularia-Test (Reis) / protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine Überlegenheit in der Wirksamkeit gegenüber dem Stand der Technik zeigen in diesem Test z. B. die Verbindungen gemäß folgender Beispiele:

**Tabelle A**

Pyricularia-Test (Reis) / protektiv

| Wirkstoffe | Wirkstoffkonzentration in % | Krankheitsbefall in % der unbehandelten Kontrolle |
|---|---|---|
| 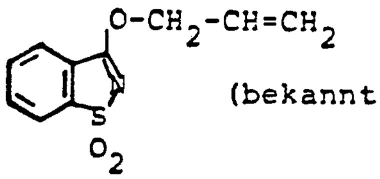 (bekannt) | 0,025 | 25 |
| 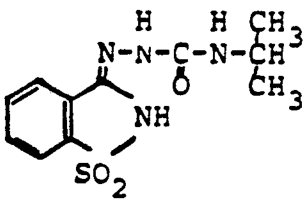 | 0,025 | 10 |
| 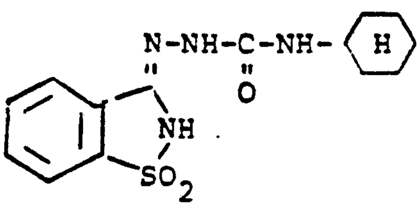 | 0,025 | 30 |
| 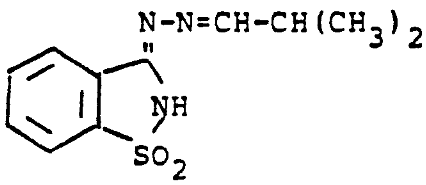 | 0,025 | 20 |
| 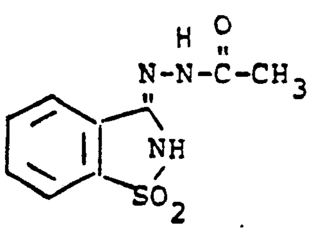 | 0,025 | 20 |

**Beispiel B**

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25° C und einer rel. Luftfeuchtigkeit von 100 % bis zur Auswertung.

4 Tage nach der Inokulation erfolgt die Auswertung des Krankheitsbefalls.

Eine Überlegenheit in der Wirksamkeit gegenüber dem Stand der Technik zeigen in diesem Test z. B. die Verbindungen gemäß folgender Beispiele:

**Tabelle B**

Pyricularia-Test (Reis) / systemisch

| Wirkstoffe | Aufwandmenge in mg Wirkstoff pro 100 $cm^3$ | Krankenheitsbefall in % der unbehandelten Kontrolle |
|---|---|---|
| 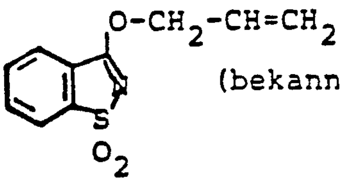 (bekannt) | 100 | 50 |
| 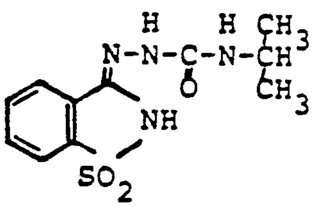 | 100 | 22 |
| 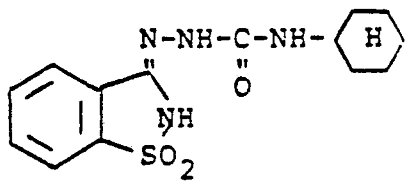 | 100 | 20 |
| 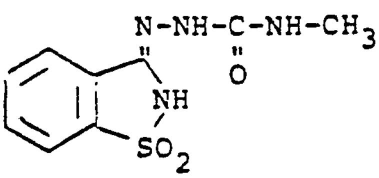 | 100 | 10 |
| 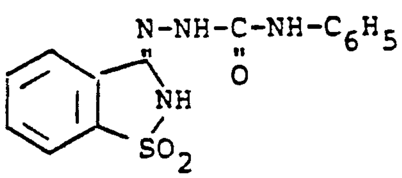 | 100 | 20 |

**Tabelle B** (Fortsetzung)

Pyricularia-Test (Reis) / systemisch

| Wirkstoffe | Aufwandmenge in mg Wirkstoff pro 100 cm³ | Krankenheitsbefall in % der unbehandelten Kontrolle |
|---|---|---|
| 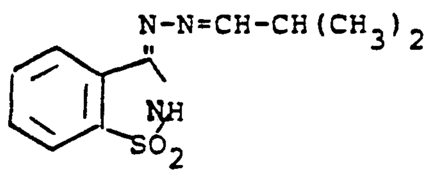 | 100 | 33 |
| 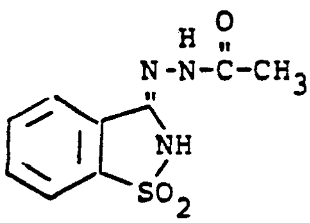 | 100 | 11 |
| 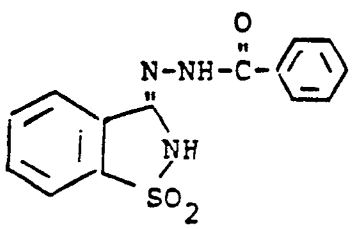 | 100 | 22 |

**Herstellungsbeispiele**

Die folgenden Beispiele sollen die Erfindung erläutern, ohne sie zu beschränken:

**Beispiel 1**

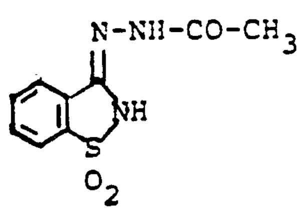

9,8 g (0,05 Mol) 3-Hydrazino-2H,3H-1,2-benzisothiazol-1,1-dioxid werden in einem Überschuß (15 g) von Acetanhydrid unter langsamer Temperatursteigerung auf 60°C gebracht. Nach 2 h wird abgekühlt, eingeengt

und der Rückstand aus Ethanol umkristallisiert. Man erhält 9,4 g (79 % der Theorie) 3-Acetylhydrazino-1,2-benzisothiazol-1,1-dioxid mit dem Schmelzpunkt 274° C.

**Beispiel 2**

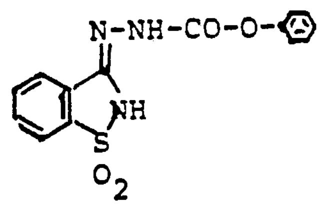

9,8 g (0,05 Mol) 3-Hydrazino-2H,3H-1,2-benzisothiazol-1,1-dioxid werden in 100 ml Dioxan mit 5 g (0,05 Mol) Triethylamin versetzt und unter Rühren anschließend 8,6 g (0,55 Mol) Phenoxycarbonylchlorid, in 25 ml Dioxan gelöst, zugetropft. Nach ca. 2 h wird das ausgefallene Salz abgesaugt, mit 15 ml kaltem Dioxan gewaschen und die vereinten Mutterlaugen eingeengt. Kristallisation aus Ethanol ergibt 12,8 g (81 % der Theorie) 3-Phenoxycarbonyl-hydrazino-2H,3H-1,2-benzisothiazol-1,1-dioxid mit dem Schmelzpunkt 216° C.

**Beispiel 3**

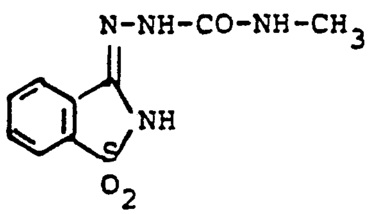

9,8 g (0,05 Mol) 3-Hydrazino-2H,3H-1,2-benzisothiazol-1,1-dioxid werden in 100 ml Dioxan bei Raumtemperatur mit einer Lösung von 3,7 g (0,55 Mol) Methylisocyanat in 30 ml Dioxan tropfenweise versetzt. Es wird 1 h nachgerührt und anschließend abgesaugt. Man erhält 11,8 g (89 % der Theorie) 3-Methylaminocarbonyl-hydrazino-1,2-benzisothiazol-1,1-dioxid mit dem Schmelzpunkt 183° C. Das Produkt kann aus Ethanol umkristallisiert werden.

Analog den Beispielen 1 bis 3 können die folgenden Verbindungen der Formel

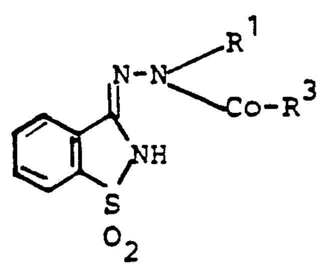

hergestellt werden:

| Beispiel-Nr. | $R^1$ | $R^3$ | Schmelzpunkt (° C) |
|---|---|---|---|
| 4 | H | $-CH_3$ | 274 |
| 5 | $CH_3$ | $-CH_3$ | 193 |
| 6 | H | $-C_2H_5$ | 211 |
| 7 | $CH_3$ | $-C_2H_5$ | 189 |
| 8 | H | $-C_3H_7$-n | 110 |
| 9 | H | $-C_6H_5$ | 272 |

| | | | |
|---|---|---|---|
| 10 | H | $-CH_2-C_6H_5$ | 232 |
| 11 | H | $-CH_2-CH_2-O-C_6H_5$ | 239 |
| 12 | H | $-O-CH_3$ | 211 |
| 13 | H | $-o-C_2H_5$ | 207 |
| 14 | $CH_3$ | $-O-C_2H_5$ | 191 |
| 15 | H | $OC_6H_5$ | 216 |
| 16 | H | -O-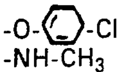-Cl | 227 |
| 17 | H | $-NH-CH_3$ | 183 |
| 18 | H | $-NH-C_3H_7$-iso | 240 |
| 19 | H | $-NH-C_6H_{11}$ | 220 |
| 20 | H | $-NH-C_6H_5$ | 208 |

**Beispiel 21**

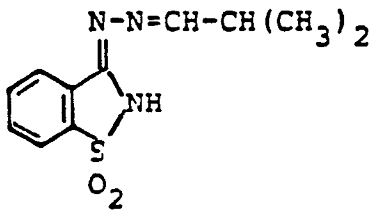

19,7 g (0,1 Mol) 3-Hydrazino-2H,3H-1,2-benzisothiazol-1,1-dioxid werden in 200 ml Dioxan bei 40 - 50°C gelöst und unter intensivem Rühren 8 g (0,11 Mol) Isobutyraldehyd zugetropft. Das Produkt fällt hierbei direkt aus. Nach Ende der Zugabe wird für ca. 20 min. auf 80 - 90°C erwärmt, anschließend abgekühlt und die Kristalle abgesaugt. Man erhält so 22,1 g (88,1 % der Theorie) 3-Iso-butylidenhydrazino-1,2-benzisothiazol-1,1-dioxid vom Schmelzpunkt 225°C.

**Beispiel 22**

Analog Beispiel 21 erhält man in einem 0,1-molaren Ansatz mit 13,2 g (0,11 Mol) 4-Methyl-benzaldehyd 24,3 g (81 % der Theorie) 3-[4-Methylbenzyliden-hydrazino]-1,2-benzisothiazol-1,1-dioxid vom Schmelzpunkt 294°C.

**Beispiel 23**

Analog Beispiel 21 erhält man mit 0,2 Mol Methacrolein in einem 0,1-molaren Ansatz 15,2 g (61 % der Theorie) 3-(2-Methylallylidenhydrazino)-1,2-benzisothiazol-1,1-dioxid vom Schmelzpunkt 224°C.

Analog den Beispielen 21 bis 23 können die folgenden Verbindungen der Formel

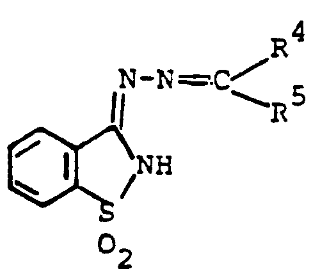

hergestellt werden:

| Beispiel-Nr. | $R^4$ | $R^5$ | Schmelzpunkt (°C) |
|---|---|---|---|
| 24 | H | $CH_2=C(CH_3)-$ | 224 |
| 25 | H | $CH_3CH=CH-$ | 196 |

| | | | |
|---|---|---|---|
| 26 | H | 2-Furyl- | 257 |
| 27 | H | $CH_3CH=C(CH_3)$- | 262 |
| 28 | H | $\Delta^3$-Cyclohexenyl | 221 |
| 29 | H | $C_6H_5CH=CH$- | 260 |
| 30 | H | $-CH(CH_3)_2$ | 225 |
| 31 | $CH_3$ | $CH_3$ | 215 |
| 32 | Cyclopentyliden | | 231 |
| 33 | Cyclohexyliden | | 177 |
| 34 | H | $3,4-Cl_2-C_6H_3$- | 321 |
| 35 | H | $3-Cl-C_6H_4$- | 306 |
| 36 | H | $4-Cl-C_6H_4$ | 315 |
| 37 | H | $3-CH_3O-C_6H_4$- | 264 |
| 38 | H | $4-CH_3O-C_6H_4$- | 281 |
| 39 | H | $4-F-C_6H_4$- | 302 |
| 40 | H | $3-F-C_6H_4$- | 318 |
| 41 | H | $4-CH_3-C_6H_4$- | 294 |
| 42 | H | $C_6H_5$- | 287 |

**Patentansprüche**

1. Fungizide Mittel, gekennzeichnet durch einen Gehalt an mindestens einem 3-Hydrazino-1,2-benzisothiazol-1,1-dioxid-Derivat der Formel (I)

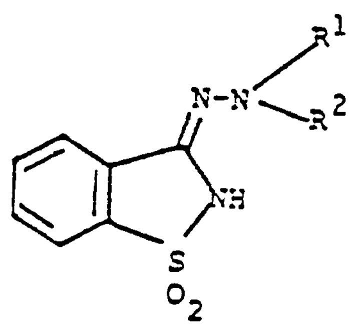

(I)

in welcher
$R^1$ für Wasserstoff, für Alkyl mit 1 bis 4 Kohlenstoffatomen, für Cycloalkyl mit 5 oder 6 Kohlenstoffatomen oder für Phenyl steht,
$R^2$ für $-CO-R^3$ steht, wobei
$R^3$ für Alkyl mit 1 bis 4 Kohlenstoffatomen, für Cyclohexyl, für Benzyl, für Phenyl, für Alkoxy mit 1 bis 4 Kohlenstoffatomen, für Phenoxymethyl, für gegebenenfalls ein- bis dreifach durch Halogen substituiertes Phenoxy, für Alkylamino mit 1 bis 4 Kohlenstoffatomen, für Cycloalkylamino mit 5 oder 6 Kohlenstoffatomen oder für Phenylamino steht, oder
$R^1$ und $R^2$ gemeinsam für eine

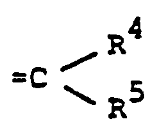

-Gruppe stehen,
worin
$R^4$ für Wasserstoff oder für Alkyl mit 1 bis 3 Kohlenstoffatomen steht,
$R^5$ für Alkyl mit 1 bis 4 Kohlenstoffatomen, für Alkenyl mit 2 bis 5 Kohlenstoffatomen, für Cycloalkenyl mit 5 oder 6 Kohlenstoffatomen, für Phenylvinyl, für Furyl, für gegebenenfalls ein bis dreifach durch Halogen, Alkyl mit 1 bis 3 Kohlenstoffatomen und Alkoxy mit 1 bis 3 Kohlenstoffatomen substituiertes Phenyl steht, oder
$R^4$ und $R^5$ für Alkylen mit 4 oder 5 Kohlenstoffatomen stehen.

2. Fungizide Mittel, gekennzeichnet durch einen Gehalt an mindestens einem 3-Hydrazino-1,2-benzisothiazol-1,1-dioxid-Derivat der Formel (I) gemäß Anspruch 1, worin
$R^1$ für Wasserstoff oder für Methyl steht,
$R^2$ für $-CO-R^3$ steht, wobei
$R^3$ für Methyl, Ethyl, n- und iso-Propyl, für Phenyl, für Benzyl, für Phenoxymethyl, für Methoxy, Ethoxy und n-und iso-Propoxy, für gegebenenfalls ein- oder zweifach durch Chlor substituiertes Phenoxy, für Methylamino, Ethylamino, n- und iso-Propylamino, für Cyclohexylamino oder Phenylamino steht, oder

$R^1$ und $R^2$ gemeinsam für eine

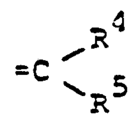

-Gruppe stehen,

worin

$R^4$ für Wasserstoff, für Methyl oder Ethyl steht,

$R^5$ für Methyl, Ethyl, n- und iso-Propyl, für Ethenyl, 1-Methyl-ethenyl, Allyl, Propenyl, 1-Methyl-Propenyl, für $\Delta^3$-Cyclohexenyl, für Phenylvinyl, für Furyl, für gegebenenfalls ein- oder zweifach durch Fluor, Chlor, Methyl, Ethyl, Methoxy und Ethoxy substituiertes Phenyl steht, oder

$R^4$ und $R^5$ für Tetra- oder Pentamethylen stehen.

3. Verwendung von 3-Hydrazino-1,2-benzisothiazol-1,1-dioxid-Derivaten der Formel (I) gemäß Anspruch 1 zur Bekämpfung von Pilzen.

4. Verfahren zur Bekämpfung von Pilzen, dadurch gekennzeichnet, daß man 3-Hydrazino-1,3-benzisothiazol-1,1-dioxid-Derivate der Formel (I) gemäß Anspruch 1 auf Pilze und/oder ihren Lebensraum einwirken läßt.

5. Verfahren zur Herstellung von fungiziden Mitteln, dadurch gekennzeichnet, daß man 3-Hydrazino-1,2-benzisothiazol-1,1-dioxid-Derivate der Formel (I) gemäß Anspruch 1 mit Streckmitteln und/oder oberflächenaktiven Mitteln vermischt.

6. 3-Hydrazino-1,2-benzisothiazol-1,1-dioxid-Derivate der Formel (IB)

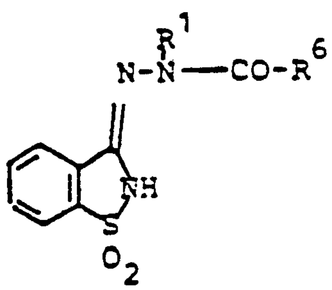

(IB)

in welcher

$R^1$ für Wasserstoff, für Alkyl mit 1 bis 4 Kohlenstoffatomen, für Cycloalkyl mit 5 oder 6 Kohlenstoffatomen oder für Phenyl steht und

$R^6$ für Alkylamino mit 1 bis 4 Kohlenstoffatomen, für Cycloalkylamino mit 5 oder 6 Kohlenstoffatomen oder für Phenylamino steht, ausgenommen die Verbindung in der $R^1$ für Wasserstoff und $R^6$ für Phenylamino stehen.

7. 3-Hydrazino-1,2-benzisothiazol-1,1-dioxid-Derivate der Formel (IB) gemäß Anspruch 6, in welcher

$R^1$ für Wasserstoff oder Methyl steht,

$R^6$ für Methylamino, Ethylamino, n- und iso-Propylamino, für Cyclohexylamino oder Phenylamino steht, ausgenommen die Verbindung, in der $R^1$ für Wasserstoff und $R^6$ für Phenylamino stehen.

8. Verfahren zur Herstellung von 3-Hydrazino-1,2-benzisothiazol-1,1-dioxid-Derivaten der Formel (IB)

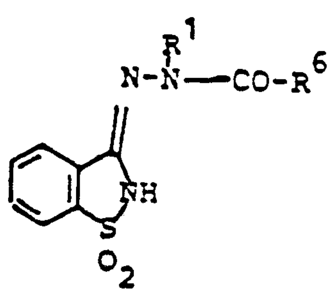

(IB)

in welcher

$R^1$ für Wasserstoff, für Alkyl mit 1 bis 4 Kohlenstoffatomen, für Cycloalkyl mit 5 oder 6 Kohlenstoffatomen oder für Phenyl steht und

$R^6$ für Alkylamino mit 1 bis 4 Kohlenstoffatomen, für Cycloalkylamino mit 5 oder 6 Kohlenstoffatomen oder für Phenylamino steht, ausgenommen die Verbindung in der $R^1$ für Wasserstoff und $R^6$ für Phenylamino stehen,

dadurch gekennzeichnet, daß man 3-Hydrazino-2H, 3H-1,2-benzisothiazol-1,1-dioxide der Formel (II)

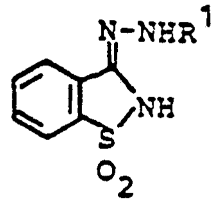

(II)

in welcher
$R^1$ die oben angegebene Bedeutung hat,
mit Isocyanaten der Formel (III)

$OCN\text{-}R^6$ (III)

in welcher
$R^6$ die oben angegebene Bedeutung hat,
gegebenenfalls in Gegenwart eines Lösungsmittels umsetzt.

## Claims

1. Fungicidal agents, characterized in that they contain at least one 3-hydrazino-1,2-benzisothiazole dioxide derivative of the formula (I)

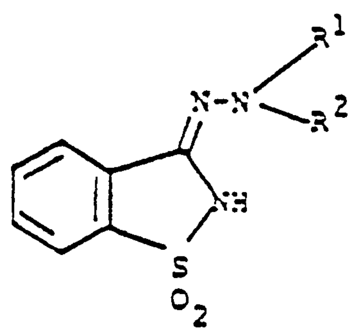

(I)

in which
$R^1$ represents hydrogen, alkyl having 1 to 4 carbon atoms, cycloalkyl having 5 or 6 carbon atoms or phenyl,
$R^2$ represents $-CO-R^3$,
wherein
$R^3$ represents alkyl having 1 to 4 carbon atoms, cyclohexyl, benzyl, phenyl, alkoxy having 1 to 4 carbon atoms or phenoxymethyl, or represents phenoxy which is optionally monosubstituted to trisubstituted by halogen, or represents alkylamino having 1 to 4 carbon atoms, cycloalkylamino having 5 or 6 carbon atoms or phenylamino, or $R^1$ and $R^2$ together represent a

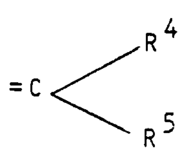

group,
wherein
$R^4$ represents hydrogen or alkyl having 1 to 3 carbon atoms,
$R^5$ represents alkyl having 1 to 4 carbon atoms, alkenyl having 2 to 5 carbon atoms, cycloalkenyl having 5 or 6 carbon atoms, phenylvinyl or furyl, or represents phenyl which is optionally monosubstituted to trisubstituted by halogen, alkyl having 1 to 3 carbon atoms and alkoxy having 1 to 3 carbon atoms, or
$R^4$ and $R^5$ represent alkylene having 4 or 5 carbon atoms.

2. Fungicidal agents, characterized in that they contain at least one 3-hydrazino-1,2-benzisothiazole 1,1-dioxide derivative of the formula (I) according to Claim 1,

wherein
$R^1$ represents hydrogen or methyl,
$R^2$ represents -CO-$R^3$,
wherein
$R^3$ represents methyl, ethyl, n- and iso-propyl, phenyl, benzyl, phenoxymethyl, methoxy, ethoxy and n- and iso-propoxy, or represents phenoxy which is optionally monosubstituted or disubstituted by chlorine, or represents methylamino, ethylamino, n- and iso-propylamino, cyclohexylamino or phenylamino, or

$R^1$ and $R^2$ together represent a 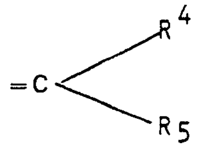 group,
wherein

$R^4$ represents hydrogen, methyl or ethyl,
$R^5$ represents methyl, ethyl, n- and iso-propyl, ethenyl, 1-methyl-ethenyl, allyl, propenyl, 1-methyl-propenyl, $\Delta^3$-cyclohexenyl, phenylvinyl or furyl, or represents phenyl which is optionally monosubstituted or disubstituted by fluorine, chlorine, methyl, ethyl, methoxy and ethoxy, or
$R^4$ and $R^5$ represent tetramethylene or pentamethylene.

3. Use of 3-hydrazino-1,2-benzisothiazole 1,1-dioxide derivatives of the formula (I) according to Claim 1 for combating fungi.

4. Method of combating fungi, characterized in that 3-hydrazino-1,3-benzisothiazole 1,1-dioxide derivatives of the formula (I) according to Claim 1 are allowed to act on fungi and/or their habitat.

5. Process for the preparation of fungicidal agents, characterized in that 3-hydrazino-1,2-benzisothiazole 1,1-dioxide derivatives of the formula (I) according to Claim 1 are mixed with extenders and/or surface-active agents.

6. 3-Hydrazino-1,2-benzisothiazole 1,1-dioxide derivatives of the formula (IB)

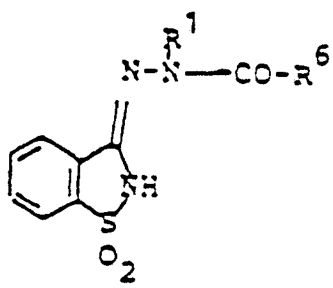

(IB)

in which
$R^1$ represents hydrogen, alkyl having 1 to 4 carbon atoms, cycloalkyl having 5 or 6 carbon atoms or phenyl and
$R^6$ represents alkylamino having 1 to 4 carbon atoms, cycloalkylamino having 5 or 6 carbon atoms or phenylamino, with the exception of the compound in which $R^1$ represents hydrogen and $R^6$ represents phenylamino.

7. 3-Hydrazino-1,2-benzisothiazole 1,1-dioxide derivatives of the formula (IB) according to Claim 6,
in which
$R^1$ represents hydrogen or methyl and
$R^6$ represents methylamino, ethylamino, n- and iso-propylamino, cyclohexylamino or phenylamino, with the exception of the compound in which $R^1$ represent hydrogen and $R^6$ represents phenylamino.

8. Process for the preparation of 3-hydrazino-1,2-benzisothiazole 1,1-dioxide derivatives of the formula (IB)

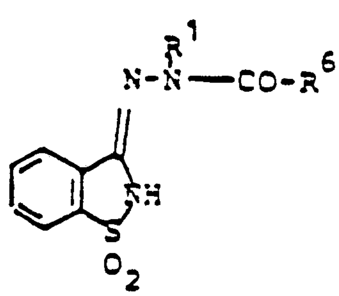

(IB)

in which

$R^1$ represents hydrogen, alkyl having 1 to 4 carbon atoms, cycloalkyl having 5 or 6 carbon atoms or phenyl and

$R^6$ represents alkylamino having 1 to 4 carbon atoms, cycloalkylamino having 5 or 6 carbon atoms or phenylamino, with the exception of the compound in which $R^1$ represents hydrogen and $R^6$ represents phenylamino,

characterized in that 3-hydrazino-2H,3H-1,2-benzisothiazole 1,1-dioxides of the formula (II)

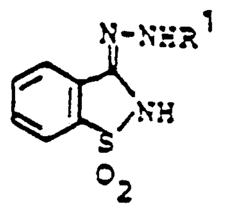

(II)

in which $R^1$ has the meaning given above,

are reacted with isocyanates of the formula (III)

OCN-$R^6$ (III)

in which

$R^6$ has the meaning given above,

if appropriate in the presence of a solvent.

**Revendications**

1. Compositions fongicides, caractérisées par une teneur en au moins un dérivé de 1,1-dioxyde de 3-hydrazino-1,2-benzisothiazole de formule (I)

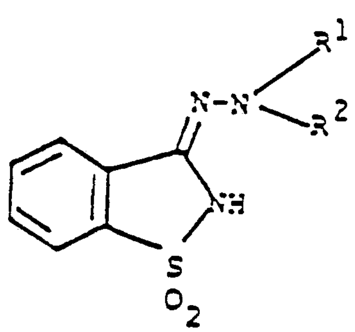

(I)

dans laquelle

$R^1$ représente l'hydrogène, un groupe alkyle ayant 1 à 4 atomes de carbone, un groupe cycloalkyle ayant 5 ou 6 atomes de carbone ou le groupe phényle,

$R^2$ est un groupe -CO-$R^3$, dans lequel

$R^3$ est un groupe alkyle ayant 1 à 4 atomes de carbone, un groupe cyclohexyle, un groupe benzyle, un groupe phényle, un groupe alkoxy ayant 1 à 4 atomes de carbone, un groupe phénoxyméthyle, un groupe phénoxy portant éventuellement un à trois substituants halogéno, un groupe alkylamino ayant 1 à 4 atomes de carbone, un groupe cycloalkylamino ayant 5 ou 6 atomes de carbone ou un groupe phénylamino, ou bien

$R^1$ et $R^2$ forment conjointement un groupe

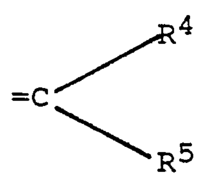

dans lequel

$R^4$ représente l'hydrogène ou un groupe ayant 1 à 3 atomes de carbone,

$R^5$ est un groupe alkyle ayant 1 à 4 atomes de carbone, un groupe alcényle ayant 2 à 5 atomes de carbone, un groupe cycloalcényle ayant 5 ou 6 atomes de carbone, un groupe phénylvinyle, un groupe furyle, un groupe phényle portant éventuellement un à trois substituants halogéno, alkyle ayant 1 à 3 atomes de carbone et alkoxy ayant 1 à 3 atomes de carbone, ou bien

$R^4$ et $R^5$ représentent un groupe alkylène ayant 4 ou 5 atomes de carbone.

2. Compositions fongicides, caractérisées par une teneur en au moins un dérivé de 1,1-dioxyde de 3-hydrazino-1,2-benzisothiazole de formule (I) suivant la revendication 1, dans laquelle

R1 est l'hydrogène ou un groupe méthyle,

$R^2$ est un groupe -CO-$R^3$, dans lequel

$R^3$ est un groupe méthyle, éthyle, n-propyle, isopropyle, phényle, benzyle, phénoxyméthyle, méthoxy, éthoxy, n-propoxy, isopropoxy, un groupe phénoxy portant éventuellement 1 ou 2 substituants chloro, un groupe méthylamino, éthylamino, n-propylamino, isopropylamino, cyclohexylamino ou phénylamino, ou bien

$R^1$ et $R^2$ forment conjointement un groupe

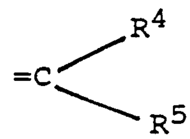

$R^4$ représente l'hydrogène, un groupe méthyle ou un groupe éthyle,

$R^5$ est un groupe méthyle, éthyle, n-propyle, isopropyle, éthényle, 1-méthyl-éthényle, allyle, propényle, 1-méthyl-propényle, $\Delta^3$-cyclohexényle, phénylvinyle, furyle, un groupe phényle portant éventuellement un ou deux substituants fluoro, chloro, méthyle, éthyle, méthoxy et éthoxy, ou bien

$R^4$ et $R^5$ représentent un groupe tétraméthylène ou pentaméthylène.

3. Utilisation de dérivés de 1,1-dioxyde de 3-hydrazino-1,2-benzisothiazole de formule (I) suivant la revendication 1 pour combattre des champignons.

4. Procédé pour combattre des champignons, caractérisé en ce qu'on fait agir des dérivés de 1,1-dioxyde de 3-hydrazino-1,3-benzisothiazole de formule (I) suivant la revendication 1 sur des champignons et/ou sur leur milieu.

5. Procédé de préparation de compositions fongicides, caractérisé en ce qu'on mélange des dérivés de 1,1-dioxyde de 3-hydrazino-1,2-benzisothiazole de formule (I) suivant la revendication 1 avec des diluants et/ou des agents tensio-actifs.

6. Dérivés de 1,1-dioxyde de 3-hydrazino-1,2-benzisothiazole de formule (IB)

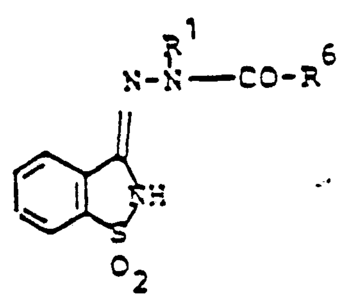

(IB)

dans laquelle

$R^1$ représente l'hydrogène, un groupe alkyle ayant 1 à 4 atomes de carbone, un groupe cycloalkyle ayant 5 ou 6 atomes de carbone ou un groupe phényle et

$R^6$ est un groupe alkylamino ayant 1 à 4 atomes de carbone, cycloalkylamino ayant 5 ou 6 atomes de carbone ou phénylamino, excepté le composé dans lequel $R^1$ est l'hydrogène et $R^6$ est le groupe phénylamino.

7. Dérivés de 1,1-dioxyde de 3-hydrazino-1,2-benzisothiazole de formule (IB) suivant la revendication 6, dans laquelle

$R^1$ est l'hydrogène ou un groupe méthyle,

$R^6$ est un groupe méthylamino, éthylamino, n-propylamino, isopropylamino, cyclohexylamino ou phénylamino, excepté le composé dans lequel $R^1$ est l'hydrogène et $R^6$ est un groupe phénylamino.

8. Procédé de préparation de dérivés de 1,1-dioxyde de 3-hydrazino-1,2-benzisothiazole de formule (IB)

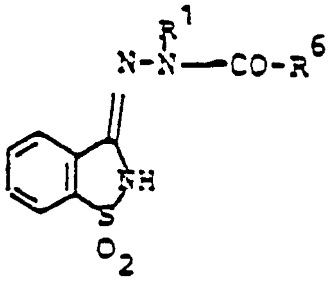

(IB)

dans laquelle

$R^1$ représente l'hydrogène, un groupe alkyle ayant 1 à 4 atomes de carbone, cycloalkyle ayant 5 ou 6 atomes de carbone ou un groupe phényle et

$R^6$ est un groupe alkylamino ayant 1 à 4 atomes de carbone, cycloalkylamino ayant 5 ou 6 atomes de carbone ou un groupe phénylamino, excepté le composé dans lequel $R^1$ est l'hydrogène et $R^6$ est un groupe phénylamino,

caractérisé en ce qu'on fait réagir des 1,1-dioxydes de 3-hydrazino-2H,3H-1,2-benzisothiazole de formule (II)

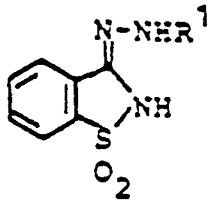

(II)

dans laquelle

$R^1$ a la définition indiquée ci-dessus,

avec des isocyanates de formule (III)

$OCN\text{-}R^6$ (III)

dans laquelle

$R^6$ a la définition indiquée ci-dessus,

le cas échéant en présence d'un solvant.